# EUROPÄISCHE PATENTSCHRIFT

(11) **EP 2 618 944 B1**
(45) Veröffentlichungstag und Bekanntmachung des Hinweises auf die Patenterteilung: **24.02.2016**
(21) Anmeldenummer: 11769774.8
(22) Anmeldetag: 21.09.2011
(51) Int. Cl.: B05C 17/005, B05C 17/015, A61B 17/88

(54) **AUSPRESSVORRICHTUNG FÜR KNOCHENZEMENT**
DISPENSER FOR BONE CEMENT
DISTRIBUTEUR DE CIMENT OSSEUX

(30) Priorität: 22.09.2010 DE 102010046058
(43) Veröffentlichungstag der Anmeldung: 31.07.2013
(73) Patentinhaber: Heraeus Medical GmbH, 61273 Wehrheim (DE)
(72) Erfinder: VOGT, Sebastian, 99092 Erfurt (DE); BUECHNER, Hubert, 90491 Nuernberg (DE)
(74) Vertreter: Schultheiss & Sterzel Patentanwälte PartG mbB
(86) Internationale Anmeldenummer: PCT/EP2011/004715
(87) Internationale Veröffentlichungsnummer: WO 2012/038073

(56) Entgegenhaltungen:
- EP-A1- 1 118 313
- US-A- 3 568 892
- US-B1- 6 264 629

## Beschreibung

Die Erfindung betrifft eine Austragsvorrichtung zum Auspressen eines Inhalts eines Behälters durch Beaufschlagung mit einem komprimierten Gas umfassend ein Gehäuse, zumindest einen Druckgasbehälter, eine Bedieneinrichtung zum Einstellen eines Gasflusses aus dem Druckgasbehälter und ein Öffnungsmittel zum Öffnen des Druckgasbehälters, wobei das Öffnungsmittel relativ zum Druckgasbehälter beweglich ist.

Die Erfindung betrifft auch ein Verfahren zur Aktivierung einer Austragsvorrichtung, bei dem ein Behälter, der ein auszupressendes Material beinhaltet, in eine Halterung der Austragsvorrichtung eingesetzt wird sowie die Verwendung einer erfindungsgemäßen Auspressvorrichtung.

Gegenstand der Erfindung ist also auch eine durch komprimiertes Gas betriebene Auspressvorrichtung für Knochenzemente aus Kartuschen oder anderen Behältern. Das komprimierte Gas wird dabei durch eine in der Auspressvorrichtung angeordnete Gaspatrone bereitgestellt. Eine Auspressvorrichtung ist eine Austragsvorrichtung bei der der Austrag durch Auspressen eines Behälters erfolgt. Der Begriff Austragsvorrichtung umfasst also auch Auspressvorrichtungen im Sinne der vorliegenden Erfindung.

Das Auspressen von Kartuschen oder anderen Behältern mit Hilfe von komprimierten Gasen ist für Klebstoffe und Dichtungsmittel seit Jahrzehnten bekannt.

Die US 2,818,999 offenbart eine Dichtungspistole, die im Griffstück eine Gaspatrone enthält. Das komprimierte Gas aus der Gaspatrone drückt nach Öffnung der Gaspatrone einen Kolben innerhalb einer Kartusche in Richtung Kartuschenkopf. Die Steuerung des Flusses der pastösen Masse erfolgt durch einen zentralen, durch die Kartusche gehenden Stab, der die Austrittsöffnung der Kartusche verschließen kann.

In der US 3,938,709 wird bereits eine Austragsvorrichtung beschrieben, bei der durch Gasdruck eine Tube, die sich innerhalb des hohlen Pistolenkörpers befindet, ausgedrückt wird.

Die EP 0 169 533 A2 offenbart ein Einspritzgerät für viskose Mittel. Bei diesem fährt nach Unterbrechung der Druckgaszufuhr das Auspressen nicht fort, weil an der Abgabeöffnung ein Einspritzsteuerventil angeordnet ist, das den Strom des viskosen Mittels unterbricht. Durch das Ventil des Abzugsgriffs kann die Gaszufuhr und gleichzeitig der Austritt des viskosen Mittels gesteuert werden. Das Einspritzsteuerventil schließt, wenn keine Beaufschlagung mit komprimiertem Gas erfolgt.

In der US 4,925,061 wird ein ähnliches System beschrieben. Dabei wird jedoch das Einspritzsteuerventil über eine Stange betätigt, die mit dem Abzugsgriff verbunden ist.

Die EP 1 118 313 A1 offenbart eine Pistole zum Auspressen von Knochenzement. Der Antrieb erfolgt hierbei ebenfalls durch eine Gaspatrone. Wesentlich ist dabei, dass dieses sehr komplexe System eine Stange aufweist, die dazu bestimmt ist, die im Austragsrohr enthaltene Zementrestmenge auszutreiben. Diese elegante technische Lösung ist für konventionelle Polymethylmethacrylat-Knochenzemente gut geeignet. Für Kartuschensysteme zur Mischung mehrerer Komponenten mit statischem Mischer ist diese Pistole jedoch nicht verwendbar. Zudem ist die Fertigung dieser Pistole sehr aufwendig.

In der US 2004/0074927 A1 wird eine Auspresspistole beschrieben, welche im Wesentlichen die gleichen Merkmale wie die in der US 4,925,061 offenbarte Auspresspistole aufweist.

In den Druckschriften US 2005/0230433 A1, US 2005/0247740 A1 und US 6,935,541 B1 werden technische Lösungen vorgeschlagen, die auf dem Erfindungsgedanken der EP 0 169 533 A2 beruhen.

Die WO 2008/109439 A1 offenbart eine gattungsgemäße, durch komprimiertes Gas betriebene Austragsvorrichtung, die ein Hydraulik-Medium verwendet, auf welche das komprimierte Gas drückt. Die Gaspatrone wird durch ein Öffnungsmittel mit einem Hohldorn geöffnet, auf den der Kopf der Gaspatrone gedrückt wird, wenn die Gaspatrone eingeschraubt wird.

Das komprimierte Gas wird bei den genannten Vorrichtungen in einer im Applikator enthaltenen Gaspatrone gespeichert. Als Gaspatronen kommen technisch übliche Kohlendioxid-Patronen mit 8 g, 12 g oder 16 g Kohlendioxid-Füllung in Betracht. Diese Patronen sind durch Metallplomben verschlossen. Durch einfaches Aufstechen mit einem Hohldorn können diese Patronen leicht geöffnet werden.

Nachteilig an den beschriebenen Austragsvorrichtungen, die mit Gaspatronen angetrieben werden, ist, dass ein Anwender die Gaspatrone auch ohne Absicht entleeren kann, bevor die Kartusche oder das Behältnis für den auszupressenden Stoff an der Austragsvorrichtung angebracht ist. Der Anwender kann also aus Versehen das komprimierte Gas ungenutzt ablassen, ohne dass der auszupressende Stoff bewegt wird. Dadurch ist eine Fehlanwendung möglich. Im schlimmsten Fall ist die Gaspatrone schon komplett geleert, bevor die Kartusche oder das Behältnis für das auszupressende Material eingesetzt wird. Dies wird bestenfalls bei der Anwendung bemerkt und die Gaspatrone muss dann zu einem ungünstigen Zeitpunkt ausgetauscht werden. Der Zeitverlust und der aufwendige Austausch können gerade in Situationen, bei denen solche Austragsvorrichtungen zum Einsatz kommen, den Arbeitsablauf empfindlich stören. Zudem verursacht die verschwendete Gaspatrone unnötige Kosten und verbraucht unnötig Ressourcen.

Für den Fall der Verwendung solcher Auspressvorrichtungen im Operationssaal zum Applizieren von Knochenzementen kann dies bedeuten, dass der Ablauf der Operation gestört wird, weil nach Präparation eines Implantatlagers der Knochenzement nicht in die Knochenkavität eingepresst werden kann. Dadurch ist es möglich, dass sich die Operationszeit verlängert und sich somit das Narkoserisiko für den Patienten erhöht.

Wenn die Kartusche noch nicht richtig eingesetzt ist, die Gaspatrone aber schon geöffnet wird, kann sich die Kartusche durch die Einwirkung des komprimierten Gases ablösen, so dass sie unbeabsichtigt durch den Operationssaal bewegt wird. Eine umherfliegende Kartusche kann sowohl für den Patienten als auch für das OP-Personal zu einer Gefährdung werden.

Die Aufgabe der Erfindung besteht also darin, die Nachteile des Stands der Technik zu überwinden. Dabei soll eine durch komprimiertes Gas betriebene Auspressvorrichtung für Kartuschen oder andere Behälter, insbesondere für Knochenzemente, entwickelt werden, die möglichst handhabungssicher ist und Möglichkeiten für Bedienfehler durch den Anwender weitgehend durch konstruktive Maßnahmen ausschließt. Insbesondere soll eine Austragsvorrichtung und ein Verfahren zum Aktivieren einer Austragsvorrichtung entwickelt werden, bei denen ein unbeabsichtigtes Entleeren und Öffnen der Gaspatrone verhindert wird.

Die Aufgabe der Erfindung wird dadurch gelöst, dass die Austragsvorrichtung eine Halterung zum Befestigen eines Behälters an der Austragsvorrichtung umfasst, wobei die Halterung mit dem Öffnungsmittel derart in Wirkverbindung steht, dass durch das Befestigen eines Behälters an der Halterung eine Bewegung des Öffnungsmittels relativ zum Druckgasbehälter erfolgt, die den Druckgasbehälter öffnet.

Dadurch wird sichergestellt, dass sich die Gaspatrone nicht öffnen lässt, wenn die Kartusche oder der Behälter noch nicht mit der Austragsvorrichtung verbunden ist. Der Begriff Behälter umfasst erfindungsgemäß neben Tuben und Schlauchbeuteln auch Kartuschen und Kartuschensysteme für Knochenzemente, die besonders bevorzugte Behälter im Sinne der vorliegenden Erfindung darstellen.

Das Einstellen eines Gasflusses im Sinne der vorliegenden Erfindung bedeutet, dass der offene Leitungsquerschnitt zwischen zumindest zwei Leitungsquerschnitten einstellbar ist. Vorzugsweise ist einer davon Null, so dass der Gasfluss unterbrochen ist, beziehungsweise der Druck aus dem Druckgasbehälter nicht weitergegeben wird. Tatsächlich kommt es für die vorliegende Erfindung darauf an, wie der Druck aus dem Druckgasbehälter an den Behälter weitergeleitet wird. Über den Gasdruck ist die Auspressgeschwindigkeit, dass heißt die Strömungsgeschwindigkeit des ausströmenden Behälterinhalts, einstellbar. Besonders bevorzugt ist, dass der Gasfluss aus dem Druckgasbehälter kontinuierlich einstellbar ist.

Bei einer erfindungsgemäßen Austragsvorrichtung kann vorgesehen sein, dass der Druckgasbehälter zumindest eine Gaspatrone, insbesondere zumindest eine CO₂-Patrone, umfasst, wobei zumindest eine der Gaspatronen durch das Öffnungsmittel zu öffnen ist.

Auch kann vorgesehen sein, dass das Öffnungsmittel in Längsrichtung der Gaspatrone verschiebbar ist.

Es ist besonders vorteilhaft, wenn vorgesehen ist, dass die Austragsvorrichtung einen Behälter aufweist und der Behälter eine Kartusche oder ein Kartuschensystem mit mehreren, insbesondere parallel angeordneten Kartuschen ist, wobei die Kartusche oder das Kartuschensystem vorzugsweise einen Knochenzement, besonders bevorzugt einen Mehrkomponenten-Knochenzement, beinhaltet.

Dabei kann vorgesehen sein, dass in der Kartusche oder dem Kartuschensystem zumindest ein darin beweglicher Förderkolben angeordnet ist, der durch den Gasdruck aus dem Druckgasbehälter antreibbar ist.

Der Förderkolben treibt dann den Inhalt der Kartuschen aus den Kartuschen aus. Die durch den Gasdruck wirkende Kraft wird über den Förderkolben auf den Kartuscheninhalt vermittelt.

Erfindungsgemäße Austragsvorrichtungen können sich auch dadurch auszeichnen, dass der Druckgasbehälter über zumindest eine Druckgasleitung mit einer Öffnung in der Halterung verbindbar ist, wobei die Bedieneinrichtung in der durch die Druckgasleitung oder Druckgasleitungen hergestellte Verbindung angeordnet ist.

Als Druckgasleitungen kommen dabei sowohl geeignet befestigte Schläuche, als auch starre Rohre, Faltentbalge und Kombinationen daraus in Frage. Die Befestigungen der Druckgasleitungen sind dabei vorzugsweise so fest mit den Anschlüssen verbunden, dass sie sich nicht durch den Gasdruck aus dem Druckgasbehälter lösen.

Dabei kann vorgesehen sein, dass die Druckgasleitung oder Druckgasleitungen zumindest einen flexiblen Bereich, insbesondere zumindest ein Schlauchstück oder Faltenbalg, umfasst oder umfassen oder aus zumindest einem flexiblen Schlauch oder Faltenbalg aufgebaut ist oder sind.

In einer Weiterbildung der Erfindung ist vorgesehen, dass die Bedieneinrichtung ein einstellbares Drosselventil umfasst, das vorzugsweise über einen Abzug bedienbar ist.

Andere Bedieneinrichtungen, wie ein Drehknopf oder ein Knebel, sind ebenfalls möglich zur Bedienung des Drosselventils.

Auch kann vorgesehen sein, dass die Austragsvorrichtung einen Griff, vorzugsweise einen Pistolengriff umfasst, an dem vorzugsweise der Abzug zum Bedienen der Bedieneinrichtung angeordnet ist.

Zur Öffnung geeigneter Behälter kann vorgesehen sein, dass das Öffnungsmittel einen Hohldorn umfasst, mit dem der Druckgasbehälter aufstechbar ist, wobei über den Hohldorn eine druckdichte Verbindung mit dem Druckgasbehälter herstellbar ist und der Hohldorn vorzugsweise mit der Druckgasleitung oder den Druckgasleitungen verbunden ist.

Dabei kann vorgesehen sein, dass der Hohldorn außen dicht mit der in den Gasdruckbehälter eingestochenen Öffnung abschließt.

Gemäß einer Ausgestaltung der Erfindung kann vorgesehen sein, dass die Halterung beweglich zum Druckgasbehälter angeordnet ist und das Öffnungsmittel fest mit der Halterung verbunden ist, wobei ein Behälter in die Halterung einsteckbar ist und die Halterung hierzu ein Rastmittel umfasst, das in ein Gegenrastmittel eines Behälters greift, und wobei der Druckgasbehälter durch die an der Halterung wirkende Kraft beim Befestigen eines Behälters in der Halterung der Austragsvorrichtung zu öffnen ist.

Dabei kann vorgesehen sein, dass der Druckgasbehälter fest mit dem Gehäuse verbunden oder verbindbar ist und die Halterung mit dem Öffnungsmittel linear beweglich zum Gehäuse am Gehäuse angeordnet ist.

Hieraus ergibt sich eine erfindungsgemäße Austragsvorrichtung mit einer verschiebbaren Halterung.

Gemäß einer alternativen Ausgestaltung der Erfindung kann vorgesehen sein, dass ein Behälter in die Halterung eindrehbar oder einschraubbar ist und die Halterung drehbar relativ zum Öffnungsmittel angeordnet ist, wobei an der Halterung, insbesondere an einem Rohr an der Halterung, das sich ins Innere des Gehäuses erstreckt, ein Gewinde angeordnet ist, das mit dem Öffnungsmittel verzahnt ist, oder eine Kurvenscheibe angeordnet ist, die in ein Gegenstück am Öffnungsmittel greift, und das Öffnungsmittel linear beweglich gegen die Halterung und den Druckgasbehälter angeordnet ist, so dass eine Drehung der Halterung beim Befestigen eines Behälters an der Halterung zu einer linearen Bewegung des Öffnungsmittels gegen den Druckgasbehälter und dadurch zur Öffnung des Druckgasbehälters führt.

Hieraus ergibt sich eine erfindungsgemäße Austragsvorrichtung mit einer drehbaren Halterung.

Dabei kann vorgesehen sein, dass der Druckgasbehälter fest mit dem Gehäuse verbunden oder verbindbar ist und die Halterung mit dem Öffnungsmittel drehbar zum Gehäuse am Gehäuse angeordnet ist, vorzugsweise drehbar um einen Winkel zwischen 5° und 360°, besonders bevorzugt zwischen 5° und 15° oder zwischen 180° ud 360°, wobei die Drehbewegung der Halterung insbesondere durch einen Anschlag und/oder eine Rastung begrenzt ist.

Es kann dabei ferner vorgesehen sein, dass an der Halterung ein Befestigungsmittel zum Befestigen eines Behälters an der Halterung angeordnet ist, insbesondere ein Innengewinde, ein Bajonettverschluss oder Zapfen, die einen Gewindeausschnitt bilden.

Des Weiteren kann vorgesehen sein, dass in der Grundposition der Halterung ohne einen Behälter das Gewinde oder die Kurvenscheibe das Öffnungsmittel fixiert und die Gaspatrone geschlossen ist und in gedrehter Position der Halterung mit einem daran befestigten Behälter das Gewinde oder die Kurvenscheibe das Öffnungsmittel so fixiert, dass der Druckgasbehälter gasdurchlässig mit einer Öffnung in der Halterung verbunden ist.

Für wiederverwendbare Austragsvorrichtungen kann vorgesehen sein, dass der Gasdruckbehälter lösbar mit einer Fixierung am Gehäuse, insbesondere im Inneren des Gehäuses, verbunden oder verbindbar ist, vorzugweise über ein Gewinde, besonders bevorzugt ein Innengewinde, an der Fixierung und ein Gegengewinde, besonders bevorzugt ein Außengewinde, am Gasdruckbehälter.

Die Fixierung kann als gemeinsames Formteil zusammen mit dem Gehäuse aufgebaut sein.

Es kann auch vorgesehen sein, dass das Öffnen des Druckgasbehälters synchron zum Einsetzen eines Behälters in die Austragsvorrichtung erfolgt.

Ferner kann vorgesehen sein, dass an der Halterung eine Öffnung und ein zylindrisches Rohr angeordnet ist, das in die Öffnung mündet und das um seine Achse drehbar oder entlang einer Achse verschiebbar gegen den Druckgasbehälter gelagert ist, wobei die Halterung vorzugsweise in einem als Hülse ausgebildeten Teil des Gehäuses drehbar und/oder verschiebbar gelagert ist.

Erfindungsgemäß kann auch vorgesehen sein, dass an der Halterung mindestens ein Dichtungsring oder eine Dichtungsscheibe angeordnet ist, mit dem oder mit der ein Behälter dicht an der Halterung mit der Austragsvorrichtung verbindbar ist.

Es kann vorgesehen sein, dass die Austragsvorrichtung zumindest teilweise aus Kunststoff aufgebaut ist, vorzugsweise das Gehäuse, die Halterung, das Rohr, das Öffnungsmittel bis auf den Hohldorn, der Abzug, der Griff, die Kurvenscheibe, das Gewinde und/oder die Fixierung.

Bezüglich eines Verfahrens wird die Aufgabe der Erfindung dadurch gelöst, dass die beim Einsetzen des Behälters aufzuwendende Kraft dazu genutzt wird, die Halterung zu drehen und/oder zu verschieben, durch die Drehung oder das Verschieben der Halterung ein Öffnungsmittel gegen einen Druckgasbehälter der Austragsvorrichtung bewegt wird und der Druckgasbehälter durch die Bewegung des Öffnungsmittels geöffnet wird.

Die Öffnung des Druckgasbehälters erfolgt dabei vorzugsweise gleichzeitig mit der festen oder zumindest druckdichten Verbindung des Behälters mit der Austragsvorrichtung oder nachdem der Behälter fest oder zumindest druckdicht mit der Austragsvorrichtung verbunden wurde. Dadurch kann sichergestellt werden, dass der Druck auch bei einer geöffneten Bedienrichtung zum Einstellen des Gasflusses aus dem Druckgasbehälter nicht vorzeitig entweicht.

Dabei kann vorgesehen sein, dass das aus dem Druckgasbehälter strömende Gas zur Halterung geleitet wird und einen Druck auf zumindest einen Bereich des Behälters ausübt.

Durch den ausgeübten Druck wird der Behälter, wenn er an einer Stelle geöffnet ist, ausgepresst.

Ferner kann vorgesehen sein, dass durch den Druck ein Förderkolben im Behälter, insbesondere in der Kartusche oder dem Kartuschensystem, vorgetrieben wird, wobei dadurch der Inhalt des Behälters aus dem Behälter ausgetrieben wird.

Es kann auch vorgesehen sein, dass der Querschnitt einer Verbindung vom Druckgasbehälter zur Halterung und dadurch ein auf den Behälter einwirkender Druck durch eine Bedieneinrichtung, insbesondere ein Drosselventil, eingestellt wird.

Zusätzlich kann vorgesehen sein, dass die Halterung bis zu einem Anschlag oder einer Rastung gedreht oder verschoben wird.

Hierdurch wird erreicht, dass dem Anwender beim Einstecken oder Eindrehen des Behälters durch den durch den Anschlag oder die Rastung stark ansteigenden Widerstand gegen das Einsetzen des Behälters angezeigt wird, dass der Behälter fertig eingesetzt wurde, beziehungsweise die Austragsvorrichtung aktiviert wurde.

Ein weiterer Verfahrensschritt kann erfindungsgemäß vorsehen, dass das Öffnungsmittel linear gegen den Druckgasbehälter verschoben wird.

Die Aufgabe wird auch gelöst durch die Verwendung einer solchen Auspressvorrichtung, insbesondere unter Anwendung eines solchen Verfahrens, zum Auspressen von pastenförmigen Einkomponenten-Polymethylmethacrylat-Knochenzementen, von pastenförmigen Zweikomponenten-Polymethylmethacrylat-Knochenzementen, von pastenförmigen Dreikomponenten-Polymethylmethacrylat-Zementen, von dentalen Abformmassen, von anorganischen Knochenzementen und/oder von Polymethylmethacrylat-Knochenzementen, vorzugsweise durch Vermischen einer Pulverkomponente und einer flüssigen Monomerkomponente.

Die Erfindung basiert darauf, dass die Austragsvorrichtung so beschaffen ist, dass der Anwender den Druckgasbehälter, wie zum Beispiel eine Gaspatrone, nicht vor dem Einsetzen der Kartusche oder des Behälters in die Austragsvorrichtung öffnen kann und ein Öffnen des Druckgasbehälters nur bei eingesetzter Kartusche oder eingesetztem Behälter möglich ist. Besonders vorteilhaft ist es, wenn das Öffnen des Druckgasbehälters synchron zum Einsetzen der Kartusche oder des Behälters in die Austragsvorrichtung erfolgt. Damit ist es praktisch unmöglich, dass komprimiertes Gas unbeabsichtigt durch vorzeitige Betätigung des Öffnungsmittels aus dem Druckgasbehälter entweichen kann, ohne dass eine Kartusche oder ein Behälter mit der Austragsvorrichtung verbunden ist. Dadurch wird vermieden, dass unbeabsichtigt das komprimierte Gas austreten kann und damit die Austragsvorrichtung unbrauchbar beziehungsweise inaktiv wird.

Aus den oben dargelegten Gründen ist es sinnvoll, dass bei einer medizinischen Anwendung von gasgetriebenen Austragsvorrichtungen die Ankopplung der Kartusche an die Austragsvorrichtung mindestens zeitgleich zur Öffnung der Gaspatrone erfolgt. Dadurch kann das unbeabsichtigte Öffnen der Gaspatrone wirksam unterbunden werden. Weiterhin ist es sinnvoll, dass die Gaspatrone erst dann geöffnet werden kann, wenn die Kartusche mechanisch sicher an der Austragsvorrichtung fixiert ist.

Die Idee der Erfindung besteht auch darin, den mechanischen Vorgang der Verbindung der Kartusche oder des Behälters mit der Austragsvorrichtung zur gleichzeitigen Öffnung des Druckgasbehälters zu nutzen. Das bedeutet, wenn der Behälter über ein Gewinde oder Zapfen in die Austragsvorrichtung beziehungsweise den Applikator gedreht oder geschraubt wird, so dreht sich die Halterung beziehungsweise das Verbindungsmittel zumindest um einige Grad mit, wobei diese Drehbewegung zum Öffnen des Druckgasbehälters genutzt wird. Es kann somit eine Öffnung des Druckgasbehälters nur beim Einsetzen des Behälters in die Austragsvorrichtung erfolgen. Wenn anstatt eines Gewindes oder Zapfens eine Rastung zur Befestigung der Kartusche oder des Behälters an der Austragsvorrichtung angeordnet ist, in die die Kartusche oder der Behälter einsteckbar ist, wird der Kraftaufwand beim Einsteckvorgang dazu genutzt, das Öffnungsmittel zu bedienen, um die Gaspatrone zu öffnen. Der Druck auf die Halterung zum Verbinden der Kartusche oder des Behälters an der Austragsvorrichtung wird dazu in eine Bewegung des Öffnungsmittels gegen den Druckgasbehälter umgesetzt. Der Druckgasbehälter ist in beiden Fällen vor einer vorzeitigen, unbeabsichtigten Öffnung geschützt und wird durch den beim Einsetzen der Kartusche oder des Behälters notwendigen Kraftaufwand, das heißt Kraft beim Einstecken des Behälters oder Drehmoment beim Eindrehen oder Einschrauben des Behälters in die Halterung, geöffnet.

Im Folgenden werden Ausführungsbeispiele der Erfindung anhand von drei schematisch dargestellten Figuren erläutert, ohne jedoch dabei die Erfindung zu beschränken. Dabei zeigt:
- Figur 1:: eine schematische Querschnittansicht einer erfindungsgemäßen Austragsvorrichtung mit drehbarer Halterung;
- Figur 2:: eine schematische Querschnittansicht eines vorderen Teils einer zweiten erfindungsgemäßen Austragsvorrichtung mit drehbarer Halterung; und
- Figur 3:: eine schematische Querschnittansicht eines vorderen Teils einer dritten erfindungsgemäßen Austragsvorrichtung mit verschiebbarer Halterung.

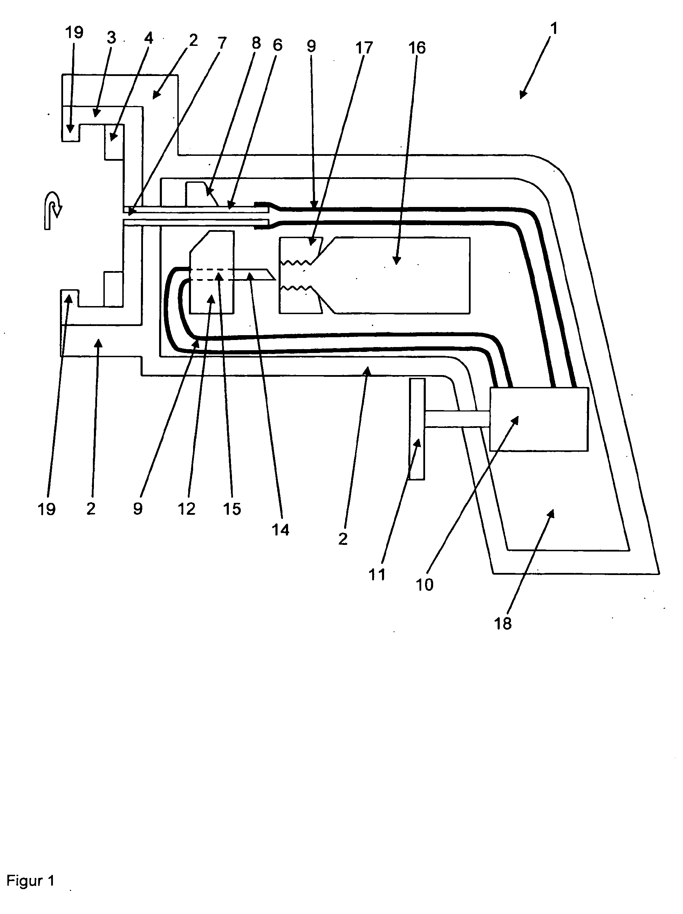

Figur 1 zeigt eine schematische Querschnittansicht einer erfindungsgemäßen Austragsvorrichtung 1. Die Austragsvorrichtung 1 umfasst ein Gehäuse 2, das beispielsweise aus Kunststoff gefertigt sein kann. Im vorderen Teil des Gehäuses 2 ist eine Halterung 3 mit einer Dichtung 4 angeordnet. Die Halterung 3 und damit auch die Dichtung 4 sind drehbar in einer durch das Gehäuse 2 gebildeten Hülse gelagert, die die Halterung 3 aufnimmt. Die Drehbarkeit der Halterung 3 ist durch den nicht ausgefüllten Pfeil angedeutet. Ein zylindrisches Rohr 6 ist mit der Halterung 3 verbunden und mündet in eine Öffnung 7 in der Halterung 3. Durch die Öffnung 7 und das Rohr 6 ist das Innere des Gehäuses 2 mit der durch das Gehäuse 2 gebildeten Hülse, beziehungsweise mit der Vorderseite der Halterung 3, verbunden. An dem Rohr 6 ist eine Kurvenscheibe 8 befestigt, die sich bei einer Drehung der Halterung 3 und damit des Rohrs 6 um die Achse des Rohrs 6 mit dreht. Die Halterung 3 ist also um die Achse, die durch das Rohr 6 gebildet wird, drehbar. Das Rohr 6 ragt ins Innere des Gehäuses 2.

Das Rohr 6 ist auf der der Öffnung 7 gegenüberliegenden Seite mit einer Gasdruckleitung 9 verbunden. Die Verbindung ist fest genug, damit sich die Gasdruckleitung 9 nicht beim Einwirken eines Gasdrucks von dem Rohr 6 löst. Dies trifft auf alle Verbindungen der Gasdruckleitung 9 mit anderen Bauteilen der Austragsvorrichtung 1 zu. In der Gasdruckleitung 9 ist ein Drosselventil 10 angeordnet, das über einen Abzug 11 einstellbar ist. Über das Drosselventil 10 ist der Querschnitt der durch die Gasdruckleitung 9 hergestellten Verbindung einstellbar. Die Gasdruckleitung 9 ist auch an einem Öffnungsmittel 12 mit einem Hohldorn 14 befestigt und bildet über eine Durchführung 15 im Öffnungsmittel 12 mit der Gasdruckleitung 9 eine weitere druckstabile Verbindung. Das Öffnungsmittel 12 ist in Richtung des Hohldorns 14 verschiebbar im Gehäuse 2 der Austragsvorrichtung 1 angeordnet. Das Öffnungsmittel 12 ist dabei derart unterhalb der Kurvenscheibe 8 des Rohrs 6 angeordnet, dass die Kurvenscheibe 8 bei einer Drehung der Halterung 3 und damit der Kurvenscheibe 8 in ein Gegenstück des Öffnungsmittels 12 greift (angedeutet durch die Schräge am linken oberen Ende des Öffnungsmittels 12). Dadurch wird das Öffnungsmittel 12 bei einer Drehung der Halterung 3 in Figur 1 nach rechts verschoben. Um die Beweglichkeit des Öffnungsmittels 12 zu gewährleisten, ist die an dem Öffnungsmittel 12 angeschlossene Gasdruckleitung 9 zumindest bereichsweise ein flexibler Schlauch oder ein Faltenbalg.

Rechts neben dem Öffnungsmittel 12 ist eine Gaspatrone 16 als Druckgasbehälter in einer Fixierung 17 am Gehäuse 2 befestigt. Die Gaspatrone 16 wird in die Fixierung 17 geschraubt. Dazu ist in der Gaspatrone 16 ein Außengewinde und in der Fixierung 17 ein Innengewinde vorgesehen. Der rechte untere Teil des Gehäuses 2 bildet einen Pistolengriff 18, an dem die Austragsvorrichtung 1 gehalten werden kann, so dass der Abzug 11 auch bei Halten mit nur einer Hand leicht bedienbar ist.

An der Halterung 3 sind Zapfen 19 vorgesehen, die einen Gewindeausschnitt bilden. In diesen Gewindeausschnitt kann ein Behälter (nicht gezeigt), zum Beispiel eine Kartusche oder ein Kartuschensystem für einen medizinischen Zement, eingeschraubt werden.

Wenn ein Behälter an der Halterung 3 befestigt wird, das heißt eingedreht beziehungsweise eingeschraubt wird, wird sich dieser bis zur Dichtung 4 eindrehen lassen. Anschließend sitzt der Behälter an der Halterung 3 fest. Bei weiterer Drehung des Behälters wird sich die Halterung 3 und damit das Rohr 6 mit der Kurvenscheibe 8 im Gehäuse 2 drehen. Die Kurvenscheibe 8 drückt mit ihrer schrägen Kante auf die schräge Kante des Gegenstücks an dem Öffnungsmittel 12. Das Öffnungsmittel 12 wird dadurch in Richtung der Gaspatrone 16 geschoben. Der Hohldorn 14 dringt in die Gaspatrone 16 ein und öffnet diese.

Das Gas aus der Gaspatrone 16 kann durch den Hohldorn 14, die Durchführung 15, die Gasdruckleitung 9 und das Drosselventil 10 in das Rohr 6 strömen. Über die Öffnung 7 gelangt das Gas aus der Gaspatrone 16 schließlich zu einem Bereich des eingeschraubten Behälters. Da der Bereich des Behälters durch die Dichtung 4 abgedichtet ist, kann sich dort ein Gasdruck aufbauen, der auf den Behälter wirkt. Durch den Druck, den das Gas auf den Bereich des Behälters ausübt, wird ein in dem Behälter enthaltenes Material aus dem Behälter ausgepresst. Die Auspressgeschwindigkeit kann durch Einstellen des Drosselventils 10 über den Abzug 11 reguliert werden.

Die Öffnung der Gaspatrone 16 erfolgt also erst, wenn der Behälter vollständig an der Austragsvorrichtung 1 angeschlossen ist. Da sich die Halterung 3 ohne den eingesetzten Behälter nicht ohne weiteres drehen lässt, ist eine Fehlbedienung der erfindunggemäßen Austragsvorrichtung 1, das heißt eine vorzeitige Öffnung der Gaspatrone 16, praktisch ausgeschlossen.

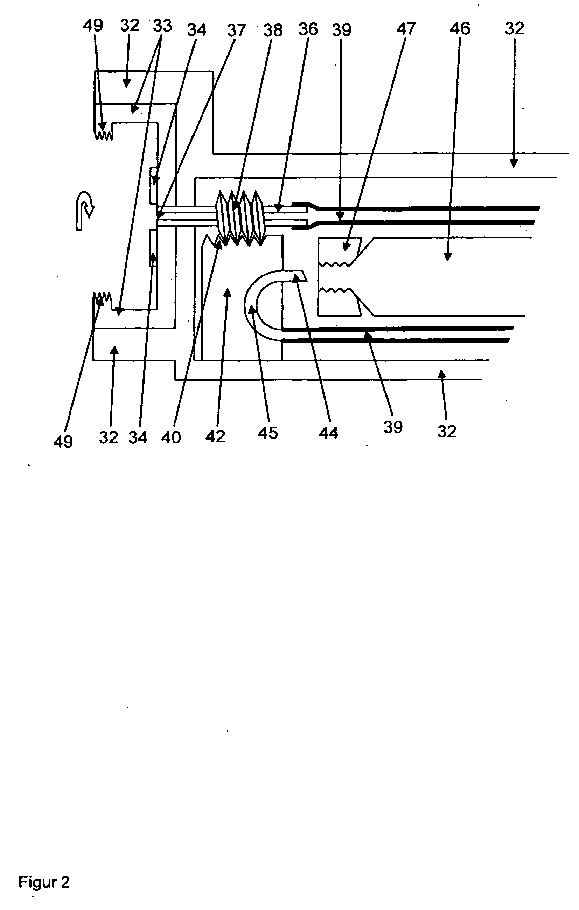

Figur 2 zeigt eine schematische Querschnittansicht eines vorderen Teils einer alternativen Austragsvorrichtung. Auch diese umfasst ein Gehäuse 32, in der eine Halterung 33 für einen Behälter (nicht gezeigt) drehbar gelagert ist. In der Halterung 33 ist eine Dichtung 34 angeordnet, an der eine flache Vorderseite eines eingesetzten Behälters anliegt, um eine gasdichte Verbindung des Behälters mit der Halterung 33 zu erzeugen. An der Halterung 33 ist Rohr 36 angeordnet, das eine Öffnung 37 in der Halterung 33 fortführt. Um das Rohr 36 herum ist ein Gewinde 38 vorgesehen. Das Gewinde 38 ist in Figur 2, im Gegensatz zu allen anderen Bauteilen der Austragsvorrichtung, nicht im Querschnitt sondern in schematischer Seitenansicht, also Aufsicht dargestellt. Das Rohr 36 verbindet die Öffnung 37 in der Halterung 33 mit einer Druckgasleitung 39. In der Druckgasleitung 39 ist ein Ventil (nicht gezeigt) zum Steuern eines Gasstroms vorgesehen.

Das Gewinde 38 des Rohrs 36 greift in ein Gegengewinde 40, das Teil eines Öffnungsmittels 42 ist, das verschiebbar im Gehäuse 32 unterhalb des Gewindes 38 angeordnet ist. Auf einer Seite des Öffnungsmittels 42 ist ein Hohldorn 44 vorgesehen, der über eine Durchführung 45 im Öffnungsmittel 42 mit der Druckgasleitung 39 verbunden ist. Die Durchführung 45 beschreibt eine Kurve im Öffnungsmittel 42, an der ein Gasstrom umleitbar ist. Der Hohldorn 44 dient dazu, eine Gaspatrone 46 zu öffnen, wenn sich das Öffnungsmittel 42 durch Drehung der Halterung 33 und damit des Gewindes 38 in Richtung der Gaspatrone 46 verschiebt. Während das Öffnungsmittel 42 im Gehäuse 32 in Längsrichtung der Gaspatrone 46 verschiebbar ist, ist die Gaspatrone 46 selbst über eine Fixierung 47 fest mit dem Gehäuse 32 verbunden. Die Gaspatrone 46 kann über ein Rastmittel, das in ein Gegenrastmittel an der Fixierung 47 greift, mit der Fixierung 47 und damit mit dem Gehäuse 32 verbunden sein.

An der Halterung 33 ist ein Befestigungsmittel in Form eines Innengewindes 49 vorgesehen, in das ein Behälter mit einem entsprechenden Außengewinde geschraubt werden kann. Sobald der Behälter bis zum Anschlag in das Befestigungsgewinde 49 geschraubt ist, führt eine weitere Drehung des Behälters zu einer Drehung der Halterung 33 und damit des Rohrs 36. Mit dem Rohr 36 dreht sich das Gewinde 38 am Rohr 36 im Inneren des Gehäuses 32. Das Gewinde 38 greift in das Gegengewinde 40 des Öffnungsmittels 42. Durch die Drehung des Gewindes 38 wird das Öffnungsmittel 42 in Richtung der Gaspatrone 46 geschoben. Der Hohldorn 44 dringt in den Kopf der Gaspatrone 46 ein und öffnet diese. Durch den Hohldorn 44 wird das komprimierte Gas aus der Gaspatrone 46 durch die Durchführung 45 in die Druckgasleitung 39 geleitet. Von dort aus wird das Gas weiter durch das Rohr 36 bis zur Öffnung 37 geleitet.

Der an der Öffnung 37 der Halterung 33 herrschende Druck wirkt auf den Behälter und wird dazu genutzt, den Inhalt des Behälters auszupressen. Die zylindrische Halterung 33 mit kreisförmiger Grundfläche sitzt in fester Passung in der durch das Gehäuse 32 gebildeten zylindrischen Hülse. Die zylindrische Halterung 33 ist bis auf das Gewinde 49 rotationssymmetrisch, wobei sich das Rohr 36 symmetrisch entlang der Symmetrieachse erstreckt. Die Drehung der Halterung 33 und des Rohrs 36 erfolgt also um die Symmetrieachse des zylindrischen Rohrs 36 (der Drehsinn ist durch den nicht ausgefüllten Pfeil angedeutet). Durch die feste Passung der Halterung 33 im Gehäuse 32 kann diese nicht ohne weiteres gedreht werden. Der einzuschraubende Behälter stellt jedoch ein geeignetes Werkzeug dar, um die Halterung 33 im Gehäuse 32 zu drehen, da über den Behälter eine ausreichende Kraftübertragung auf die Halterung 33 ausgeübt werden kann, um die Haftreibung der festen Passung zu überwinden. Dadurch wird sichergestellt, dass eine Drehung der Halterung 33 gegen das Gehäuse 32 nur mit eingesetztem Behälter erfolgen kann. Da das Öffnungsmittel 42, das hier ein verschiebbarer Körper mit dem Hohldorn 44 und mit einer an dem Hohldorn 44 angeschlossenen Durchführung 45 ist, nur durch den Antrieb über das Gewinde 38 am Rohr 36 der Halterung 33 bewegt werden kann, kann der Hohldorn 44 nur bei eingesetztem Behälter in die Gaspatrone 46 gestochen werden. Dementsprechend kann der Druckgasbehälter 46, beziehungsweise die Gaspatrone 46 auch nur bei einem in die Austragsvorrichtung eingesetzten Behälter geöffnet werden.

Für beide Ausführungsbeispiele nach den Figuren 1 und 2 gilt, dass das Drehmoment, das durch die Drehung des fixierten Behälters auf die Halterung 3, 33 übertragen wird, sich über die Kurvenscheibe 8 oder das Gewinde 38 und die Gegenmittel 40 auf dem Öffnungsmittel 12, 42 auf das Öffnungsmittel 12, 42 überträgt und durch die Geometrie der Anordnung (die Form der Kurvenscheibe 8 und der Gegenkurvenscheibe sowie des Gewindes 38 und des Gegengewindes 40) in eine lineare Bewegung des Öffnungsmittels 12, 42 gegen den Druckgasbehälter 16, 46 umgewandelt wird. Die Kraft dieser Bewegung des Öffnungsmittels 12, 42 wird zum Öffnen des Druckgasbehälters 16, 46 verwendet. Dabei kann durch geeignete Wahl der Steigung der Kante der Kurvenscheibe 8 oder des Gewindes 38 eine gewünschte Verstärkung der Kraftübertragung erzielt werden, das heißt das dadurch gebildete Getriebe eingestellt werden.

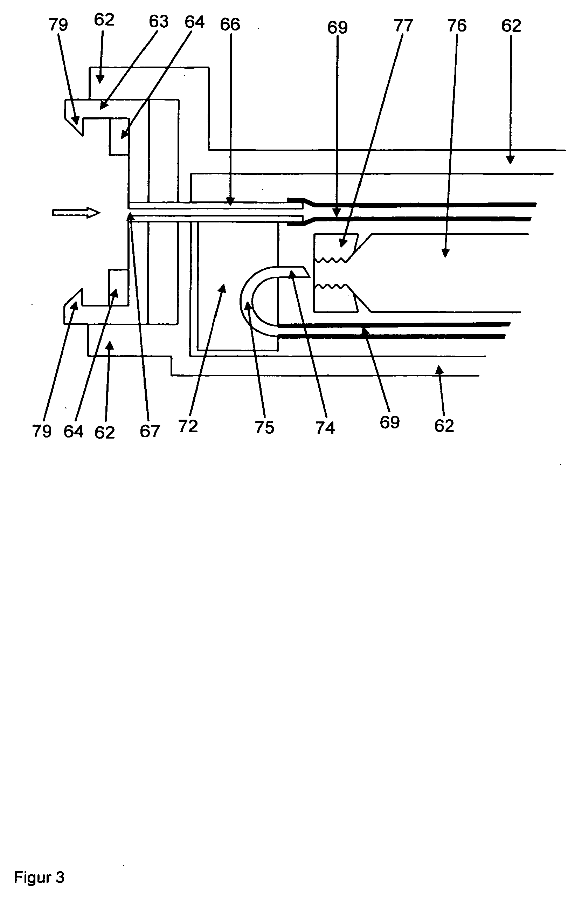

Figur 3 zeigt eine schematische Querschnittansicht eines vorderen Teils einer weiteren erfindungsgemäßen Austragsvorrichtung. Bei dieser Ausführungsform ist in einem Gehäuse 62 eine darin verschiebbare Halterung 63 für einen Behälter, wie zum Beispiel eine Kartusche oder ein Kartuschensystem, angeordnet. Das Einsetzen der Kartusche beziehungsweise des Behälters in die Halterung 63 erfolgt durch Einstecken in Richtung des nicht ausgefüllten Pfeils. Eine Dichtung 64 ist in der Halterung 63 um die Verbindung zu einem Rohr 66 herum angeordnet. Das Rohr 66 mündet in eine Öffnung 67 in der Halterung 63. Das Rohr 66 ist an eine Druckgasleitung 69 angeschlossen. In der Druckgasleitung 69 ist ein Ventil (nicht gezeigt) zum Regeln des freien Querschnitts der Druckgasleitung 69 angeordnet.

Ein Öffnungsmittel 72 ist fest mit dem Rohr 66 verbunden. Das Öffnungsmittel 72 umfasst einen Hohldorn 74, der über einen Durchgang 75 mit der Druckgasleitung 69 verbunden ist. Bei einem Verschieben der Halterung 63 in Richtung des nicht ausgefüllten Pfeils verschiebt sich auch das Rohr 66 und damit das am Rohr 66 befestigte Öffnungsmittel 72. Der Hohldorn 74 wird bei dieser Bewegung in einen Druckgasbehälter 76 gedrückt, der dadurch geöffnet wird. Der Druckgasbehälter 76 ist über ein Gewinde an einer Fixierung 77 befestigt, die fest mit dem Gehäuse 62 verbunden ist. Zum Halten des Behälters in der Halterung 63 ist ein Rastmittel 79 an der Halterung 63 vorgesehen, die in ein Gegenrastmittel am Behälter greift. Der Behälter wird also einfach durch Drücken des Behälters in die Halterung 63 mit der Austragsvorrichtung verbunden.

Durch den beim Einsetzen des Behälters ausgeübten Druck auf die Halterung 63, wird diese in Richtung des nicht ausgefüllten Pfeils im Gehäuse 62 der Austragsvorrichtung verschoben. Dadurch wird das Rohr 66 tiefer ins Innere des Gehäuses 32 hinein gedrückt. Hierdurch wird das Öffnungsmittel 72 in Richtung des Druckgasbehälters 76 verschoben. Der Hohldorn 74 dringt in eine dafür vorgesehene Schwachstelle am Kopf des Druckgasbehälters 76 ein und öffnet diesen. Der Druck aus dem Druckgasbehälter 76 wird über den Hohldorn 74, die Durchführung 75, die Druckgasleitungen 69 und das Rohr 66 zum in der Halterung 63 befestigten Behälter geleitet. Der Druck kann dazu genutzt werden, den Inhalt des Behälters auszupressen.

Die in der voranstehenden Beschreibung, sowie den Ansprüchen, Figuren und Ausführungsbeispielen offenbarten Merkmale der Erfindung können sowohl einzeln, als auch in jeder beliebigen Kombination für die Verwirklichung der Erfindung in ihren verschiedenen Ausführungsformen wesentlich sein.

### Bezugszeichenliste

- 1: Austragsvorrichtung
- 2, 32, 62: Gehäuse
- 3, 33, 63: Halterung
- 4, 34, 64: Dichtung
- 6, 36, 66: Rohr
- 7, 37, 67: Öffnung
- 8: Kurvenscheibe
- 9, 39, 69: Leitung
- 10: Drosselventil
- 11: Abzug
- 12, 42, 72: Öffnungsmittel
- 14, 44, 74: Hohldorn
- 15, 45, 75: Durchführung
- 16, 46, 76: Gaspatrone
- 17, 47, 77: Fixierung
- 18: Griff
- 19: Zapfen
- 38: Gewinde
- 40: Gegengewinde
- 49: Gewinde
- 79: Rastmittel

## Patentansprüche

1. Austragsvorrichtung zum Auspressen eines Inhalts eines Behälters durch Beaufschlagung mit einem komprimierten Gas umfassend ein Gehäuse (2, 32, 62), zumindest einen Druckgasbehälter (16, 46, 76), eine Bedieneinrichtung (10, 11) zum Einstellen eines Gasflusses aus dem Druckgasbehälter (16, 46, 76) und ein Öffnungsmittel (12, 42, 72) zum Öffnen des Druckgasbehälters(16, 46, 76), wobei das Öffnungsmittel (12, 42, 72) relativ zum Druckgasbehälter (16, 46, 76) beweglich ist und die Austragsvorrichtung (1) eine Halterung (3, 33, 63) zum Befestigen eines Behälters an der Austragsvorrichtung (1) umfasst, **dadurch gekennzeichnet, dass**
die Halterung (3, 33, 63) mit dem Öffnungsmittel (12, 42, 72) derart in Wirkverbindung steht, dass durch das Befestigen eines Behälters an der Halterung (3, 33, 63) eine Bewegung des Öffnungsmittels (12, 42, 72) relativ zum Druckgasbehälter (16, 46, 76) erfolgt, die den Druckgasbehälter (16, 46, 76) öffnet.

2. Austragsvorrichtung nach Anspruch 1, **dadurch gekennzeichnet, dass**
der Druckgasbehälter (16, 46, 76) zumindest eine Gaspatrone (16, 46), insbesondere zumindest eine CO₂-Patrone, umfasst, wobei zumindest eine der Gaspatronen (16, 46) durch das Öffnungsmittel (12, 42, 72) zu öffnen ist und das Öffnungsmittel (12, 42, 72) in Längsrichtung der Gaspatrone (16, 46) verschiebbar ist.

3. Austragsvorrichtung nach Anspruch 1 oder 2, **dadurch gekennzeichnet, dass**
die Austragsvorrichtung einen Behälter aufweist und der Behälter eine Kartusche oder ein Kartuschensystem mit mehreren, insbesondere parallel angeordneten Kartuschen ist, wobei die Kartusche oder das Kartuschensystem vorzugsweise einen Knochenzement, besonders bevorzugt einen Mehrkomponenten-Knochenzement, beinhaltet.

4. Austragsvorrichtung nach Anspruch 3, **dadurch gekennzeichnet, dass**
in der Kartusche oder dem Kartuschensystem zumindest ein darin beweglicher Förderkolben angeordnet ist, der durch den Gasdruck aus dem Druckgasbehälter (16, 46, 76) antreibbar ist.

5. Austragsvorrichtung nach einem der vorangehenden Ansprüche, **dadurch gekennzeichnet, dass**
der Druckgasbehälter (16, 46, 76) über zumindest eine Druckgasleitung (9, 39, 69) mit einer Öffnung in der Halterung (3, 33, 63) verbindbar ist, wobei die Bedieneinrichtung (10, 11) in der durch die Druckgasleitung (9, 39, 69) oder Druckgasleitungen (9, 39, 69) hergestellte Verbindung angeordnet ist.

6. Austragsvorrichtung nach Anspruch 5, **dadurch gekennzeichnet, dass**
die Druckgasleitung (9, 39, 69) oder Druckgasleitungen (9, 39, 69) zumindest einen flexiblen Bereich, insbesondere zumindest ein Schlauchstück, umfasst oder umfassen oder aus zumindest einem flexiblen Schlauch aufgebaut ist oder sind.

7. Austragsvorrichtung nach einem der vorangehenden Ansprüche, **dadurch gekennzeichnet, dass**
die Bedieneinrichtung (10, 11) ein einstellbares Drosselventil (10) umfasst, das vorzugsweise über einen Abzug (11) bedienbar ist.

8. Austragsvorrichtung nach einem der vorangehenden Ansprüche, **dadurch gekennzeichnet, dass**
die Austragsvorrichtung (1) einen Griff (18), vorzugsweise einen Pistolengriff (18), umfasst, an dem vorzugsweise der Abzug (11) zum Bedienen der Bedieneinrichtung (10, 11) angeordnet ist.

9. Austragsvorrichtung nach einem der vorangehenden Ansprüche, **dadurch gekennzeichnet, dass**
das Öffnungsmittel (12, 42, 72) einen Hohldorn (14, 44, 74) umfasst, mit dem der Druckgasbehälter (16, 46, 76) aufstechbar ist, wobei über den Hohldorn (14, 44, 74) eine druckdichte Verbindung mit dem Druckgasbehälter (16, 46, 76) herstellbar ist und der Hohldorn (14, 44, 74) vorzugsweise mit der Druckgasleitung (9, 39, 69) oder den Druckgasleitungen (9, 39, 69) verbunden ist.

10. Austragsvorrichtung nach einem der vorangehenden Ansprüche, **dadurch gekennzeichnet, dass**
die Halterung (63) beweglich zum Druckgasbehälter (76) angeordnet ist und das Öffnungsmittel (72) fest mit der Halterung (63) verbunden ist, wobei ein Behälter in die Halterung (63) einsteckbar ist und die Halterung (63) hierzu ein Rastmittel (79) umfasst, das in ein Gegenrastmittel eines Behälters greift, und wobei der Druckgasbehälter (76) durch die an der Halterung (63) wirkende Kraft beim Befestigen eines Behälters in der Halterung (63) der Austragsvorrichtung (1) zu öffnen ist.

11. Austragsvorrichtung nach Anspruch 10, **dadurch gekennzeichnet, dass**
der Druckgasbehälter (76) fest mit dem Gehäuse (62) verbunden oder verbindbar ist und die Halterung (63) mit dem Öffnungsmittel (72) linear beweglich zum Gehäuse (62) am Gehäuse (62) angeordnet ist.

12. Austragsvorrichtung nach einem der Ansprüche 1 bis 9, **dadurch gekennzeichnet, dass**
ein Behälter in die Halterung (3, 33) eindrehbar oder einschraubbar ist und die Halterung (3, 33) drehbar relativ zum Öffnungsmittel (12, 42) angeordnet ist, wobei an der Halterung (3, 33), insbesondere an einem Rohr (6, 36) an der Halterung (3, 33), das sich ins Innere des Gehäuses (2, 32) erstreckt, ein Gewinde (38) angeordnet ist, das mit dem Öffnungsmittel (12, 42) verzahnt ist, oder eine Kurvenscheibe (8) angeordnet ist, die in ein Gegenstück am Öffnungsmittel (12, 42) greift, und das Öffnungsmittel (12, 42) linear beweglich gegen die Halterung (3, 33) und den Druckgasbehälter (16, 46) angeordnet ist, so dass eine Drehung der Halterung (3, 33) beim Befestigen eines Behälters an der Halterung (3, 33) zu einer linearen Bewegung des Öffnungsmittels (12, 42) gegen den Druckgasbehälter (16, 46) und dadurch zur Öffnung des Druckgasbehälters (16, 46) führt.

13. Austragsvorrichtung nach Anspruch 12, **dadurch gekennzeichnet, dass**
der Druckgasbehälter (16, 46) fest mit dem Gehäuse (2, 32) verbunden oder verbindbar ist und die Halterung (3, 33) mit dem Öffnungsmittel (12, 42) drehbar zum Gehäuse (2, 32) am Gehäuse (2, 32) angeordnet ist, vorzugsweise drehbar um einen Winkel zwischen 5° und 360°, besonders bevorzugt zwischen 5°und 15° oder zwischen 180° und 360°, wobei die Drehbewegung der Halterung (3, 33) insbesondere durch einen Anschlag und/oder eine Rastung begrenzt ist.

14. Austragsvorrichtung nach Anspruch 12 oder 13, **dadurch gekennzeichnet, dass**
an der Halterung (3, 33) ein Befestigungsmittel (19, 39) zum Befestigen eines Behälters an der Halterung (3, 33) angeordnet ist, insbesondere ein Innengewinde (49), ein Bajonettverschluss oder Zapfen (19), die einen Gewindeausschnitt bilden.

15. Austragsvorrichtung nach einem der Ansprüche 12 bis 14, **dadurch gekennzeichnet, dass**
in der Grundposition der Halterung (3, 33) ohne einen Behälter das Gewinde (38) oder die Kurvenscheibe (8) das Öffnungsmittel (12, 42) fixiert und der Druckgasbehälter (16, 46) geschlossen ist und in gedrehter Position der Halterung (3, 33) mit einem daran befestigten Behälter das Gewinde (38) oder die Kurvenscheibe (8) das Öffnungsmittel (12, 42) so fixiert, dass der Druckgasbehälter (16, 46) gasdurchlässig mit einer Öffnung (7, 37) in der Halterung (3, 33) verbunden ist.

16. Austragsvorrichtung nach einem der vorangehenden Ansprüche, **dadurch gekennzeichnet, dass**
der Druckgasbehälter (16, 46, 76) lösbar mit einer Fixierung (12, 42, 72) am Gehäuse (2, 32, 62), insbesondere im Inneren des Gehäuses (2, 32, 62), verbunden oder verbindbar ist, vorzugweise über ein Gewinde, besonders bevorzugt ein Innengewinde, an der Fixierung (12, 42, 72) und ein Gegengewinde, besonders bevorzugt ein Außengewinde, am Druckgasbehälter (16, 46, 76).

17. Austragsvorrichtung nach einem der vorangehenden Ansprüche, **dadurch gekennzeichnet, dass**
das Öffnen des Druckgasbehälters (16, 46, 76) synchron zum Einsetzen eines Behälters in die Austragsvorrichtung (1) erfolgt.

18. Austragsvorrichtung nach einem der vorangehenden Ansprüche, **dadurch gekennzeichnet, dass**
an der Halterung (3, 33, 63) eine Öffnung (7, 37, 67) und ein zylindrisches Rohr (6, 36, 66) angeordnet ist, das in die Öffnung (7, 37, 67) mündet und das um seine Achse drehbar oder entlang einer Achse verschiebbar gegen den Druckgasbehälter (16, 46, 76) gelagert ist, wobei die Halterung (3, 33, 63) vorzugsweise in einem als Hülse ausgebildeten Teil des Gehäuses (2, 32, 62) drehbar und/oder verschiebbar gelagert ist.

19. Austragsvorrichtung nach einem der vorangehenden Ansprüche, **dadurch gekennzeichnet, dass**
an der Halterung (3, 33, 63) mindestens ein Dichtungsring (4, 34, 64) oder eine Dichtungsscheibe (4, 34, 64) angeordnet ist, mit dem oder mit der ein Behälter dicht an der Halterung (3, 33, 63) mit der Austragsvorrichtung (1) verbindbar ist.

20. Verfahren zur Aktivierung einer Austragsvorrichtung, insbesondere nach einem der vorangehenden Ansprüchen, bei dem ein Behälter, der ein auszupressendes Material beinhaltet, in eine Halterung der Austragsvorrichtung eingesetzt wird, **dadurch gekennzeichnet, dass**
die beim Einsetzen des Behälters aufzuwendende Kraft dazu genutzt wird, die Halterung zu drehen und/oder zu verschieben,
durch die Drehung oder das Verschieben der Halterung ein Öffnungsmittel gegen einen Druckgasbehälter der Austragsvorrichtung bewegt wird und
der Druckgasbehälter durch die Bewegung des Öffnungsmittels geöffnet wird.

21. Verfahren nach Anspruch 20, **dadurch gekennzeichnet, dass**
das aus dem Druckgasbehälter strömende Gas zur Halterung geleitet wird und einen Druck auf zumindest einen Bereich des Behälters ausübt.

22. Verfahren nach Anspruch 21, **dadurch gekennzeichnet, dass**
durch den Druck ein Förderkolben im Behälter, insbesondere in der Kartusche oder dem Kartuschensystem, vorgetrieben wird, wobei dadurch der Inhalt des Behälters aus dem Behälter ausgetrieben wird.

23. Verfahren nach einem der Ansprüche 20 bis 22, **dadurch gekennzeichnet, dass**
der Querschnitt einer Verbindung vom Druckgasbehälter zur Halterung und dadurch ein auf den Behälter einwirkender Druck durch eine Bedieneinrichtung, insbesondere ein Drosselventil, eingestellt wird.

24. Verfahren nach einem der Ansprüche 20 bis 23, **dadurch gekennzeichnet, dass**
die Halterung bis zu einem Anschlag oder einer Rastung gedreht oder verschoben wird.

25. Verfahren nach einem der Ansprüche 20 bis 24, **dadurch gekennzeichnet, dass**
das Öffnungsmittel linear gegen den Druckgasbehälter verschoben wird.

26. Verwendung einer Auspressvorrichtung nach einem der Ansprüche 1 bis 19, insbesondere unter Anwendung eines Verfahrens nach einem der Ansprüche 20 bis 25, zum Auspressen von pastenförmigen Einkomponenten-Polymethylmethacrylat-Knochenzementen, von pastenförmigen Zweikomponenten-Polymethylmethacrylat-Knochenzementen, von pastenförmigen Dreikomponenten-Polymethylmethacrylat-Zementen, von dentalen Abformmassen, von anorganischen Knochenzementen und/oder von Polymethylmethacrylat-Knochenzementen, vorzugsweise durch Vermischen einer Pulverkomponente und einer flüssigen Monomerkomponente.

## Claims

1. A dispensing device for pressing out a content of a container by applying a compressed gas, said device comprising a housing (2, 32, 62), at least one compressed gas container (16, 46, 76), an operating facility (10, 11) for adjusting a gas flow from the compressed gas container (16, 46, 76), and an opening means (12, 42, 72) for opening the compressed gas container (16, 46, 76), wherein the opening means (12, 42, 72) is movable relative to the compressed gas container (16, 46, 76) and the dispensing device (1) comprises a holder (3, 33, 63) for attaching a container to the dispensing device (1), **characterised in that**
the holder (3, 33, 63) and the opening means (12, 42, 72) are in operative connection such that attaching a container to the holder (3, 33, 63) moves the opening means (12, 42, 72) relative to the compressed gas container (16, 46, 76), thus opening the compressed gas container (16, 46, 76).

2. The dispensing device according to Claim 1, **characterised in that**
the compressed gas container (16, 46, 76) comprises at least one gas cartridge (16, 46), in particular at least one CO₂ cartridge, wherein at least one of the gas cartridges (16, 46) can be opened by the opening means (12, 42, 72) and the opening means (12, 42, 72) is displaceable in the longitudinal direction of the gas cartridge (16,46).

3. The dispensing device according to Claim 1 or 2, **characterised in that**
the dispensing device has a container and the container is a cartridge or a cartridge system having multiple cartridges, in particular arranged in parallel, wherein the cartridge or the cartridge system preferably contains a bone cement, particularly preferably a multi-component bone cement.

4. The dispensing device according to Claim 3, **characterised in that**
the cartridge or the cartridge system has at least one feed plunger arranged movably therein that can be driven by means of the gas pressure from the compressed gas container (16, 46, 76).

5. The dispensing device according to one of the preceding claims, **characterised in that**
the compressed gas container (16, 46, 76) is connectable via at least one compressed gas conduit (9, 39, 69) to an opening in the holder (3, 33, 63), wherein the operating facility (10, 11) is arranged in the connection established by the compressed gas conduit (9, 39, 69) or compressed gas conduits (9, 39, 69).

6. The dispensing device according to Claim 5, **characterised in that**
the compressed gas conduit (9, 39, 69) or compressed gas conduits (9, 39, 69) comprises or comprise at least one flexible region, in particular at least one hose piece, or is or are made of at least one flexible hose.

7. The dispensing device according to one of the preceding claims, **characterised in that**
the operating facility (10, 11) comprises an adjustable throttle valve (10) that preferably is operable via a trigger (11).

8. The dispensing device according to one of the preceding claims, **characterised in that**
the dispensing device (1) comprises a handle (18), preferably a pistol grip (18), on which preferably a trigger (11) for operating the operating facility (10, 11) is arranged.

9. The dispensing device according to one of the preceding claims, **characterised in that**
the opening means (12, 42, 72) comprises a hollow mandrel (14, 44, 74) by which the compressed gas container (16, 46, 76) can be punctured, wherein a pressure-tight connection to the compressed gas container (16, 46, 76) can be established via the hollow mandrel (14, 44, 74) and the hollow mandrel (14, 44, 74) preferably is connected to the compressed gas conduit (9, 39, 69) or the compressed gas conduits (9, 39, 69).

10. The dispensing device according to one of the preceding claims, **characterised in that**
the holder (63) is arranged so as to be movable with respect to the compressed gas container (76) and the opening means (72) is connected fixedly to the holder (63), wherein a container can be inserted into the holder (63) and the holder (63) for this purpose comprising a locking means (79) that engages with a counter locking means of a container, and wherein the compressed gas container (76) can be opened by the force acting on the holder (63) when the container is being attached in the holder (63) of the dispensing device (1).

11. The dispensing device according to Claim 10, **characterised in that**
the compressed gas container (76) is fixedly connected or can be fixedly connected to the housing (62) and the holder (63) having the opening means (72) is arranged on the housing (62) so as to be linearly movable with respect to the housing (62).

12. The dispensing device according to one of Claims 1 to 9, **characterised in that**
the container can be rotated or screwed into the holder (3, 33) and the holder (3, 33) is arranged so as to be rotatable relative to the opening means (12, 42), wherein a thread or a cam disk (8) is arranged on the holder (3, 33), in particular on a tube (6, 36) on the holder (3, 33) extending into the inside of the housing (2, 32), said thread being interlocked with the opening means (12, 42) or said cam disk (8) engages with a counterpart on the opening means (12, 42), and the opening means (12, 42) is arranged so as to be linearly movable with respect to the holder (3, 33) and the compressed gas container (16, 46) such that a rotation of the holder (3, 33) when the container is being attached to the holder (3, 33) leads to a linear movement of the opening means (12, 42) with respect to the compressed gas container (16, 46) and thus to the compressed gas container (16, 46) being opened.

13. The dispensing device according to Claim 12, **characterised in that**
the compressed gas container (16, 46) is fixedly connected or connectable to the housing (2, 32) and the holder (3, 33) having the opening means (12, 42) is arranged on the housing (2, 32) so as to be rotatable rotate with respect to the housing (2, 32), preferably rotatable by an angle between 5° and 360°, particularly preferably between 5° and 15°, or between 180° and 350°, wherein the rotary motion of the holder (3, 33) is limited in particular by a stop and/or a locking device.

14. The dispensing device according to Claim 12 or 13, **characterised in that**
an attaching means (19, 39) for attaching the container to the holder (3, 33) is arranged on the holder (3, 33), in particular an internal thread (49), a bayonet closure or pins (19) forming a thread section.

15. The dispensing device according to one of Claims 12 to 14, **characterised in that**,
while the holder (3, 33) is in its basic position in the absence of a container, the thread (38) or cam disk (8) fixes the opening means (12, 42) in place and the compressed gas container (16, 46) is closed and, while the holder (3, 33) is in its rotated position having a container attached to it, the thread (38) or cam disk (8) fixes the opening means (12, 42) in place such that the compressed gas container (16, 46) is connected to an opening (7, 37) in the holder (3, 33) in a gas-permeable manner.

16. The dispensing device according to one of the preceding claims, **characterised in that**
the compressed gas container (16, 46, 76) is connected or can be connected in a detachable manner to a fixation (12, 42, 72) on the housing (2, 32, 62), preferably via a thread, particularly preferably via an internal thread, on the fixation (12, 42, 72) and a counterthread, particularly preferably an external thread, on the compressed gas container (16, 46, 76).

17. The dispensing device according to one of the preceding claims, **characterised in that**
the compressed gas container (16, 46, 76) is opened synchronously with the insertion of a container into the dispensing device (1).

18. The dispensing device according to one of the preceding claims, **characterised in that**
an opening (7, 37, 67) and a cylindrical tube (6, 36, 66) are arranged on the holder (3, 33, 63), said tube (6, 36, 66) leading into the opening (7, 37, 67) and being supported such that it can be rotated about its axis or displaced along an axis with respect to the compressed gas container (16, 46, 76), wherein preferably the holder (3, 33, 63) is supported such that it can be rotated and/or displaced in a part of the housing (2, 32, 62) that is formed as a sleeve.

19. The dispensing device according to one of the preceding claims, **characterised in that**
at least one sealing ring (4, 34, 64) or a sealing washer (4, 34, 64) is arranged on the holder (3, 33, 63) and can be used to connect the container tightly to the holder (3, 33, 63) having the dispensing device (1).

20. A method for activating a dispensing device, in particular according to one of the preceding claims, in which method a container containing a material to be pressed out is inserted into a holder of the dispensing device, **characterised in that**
the force to be applied while the container is being inserted is utilised to rotate and/or displace the holder,
the rotation or displacement of the holder moves an opening means with respect to a compressed gas container of the dispensing device, and
the compressed gas container is opened by the movement of the opening means.

21. The method according to Claim 20, **characterised in that** the gas flowing from the compressed gas container is guided to the holder and a pressure is exerted onto at least a region of the container.

22. The method according to Claim 21, **characterised in that** the pressure propels a feed plunger in the container, in particular in the cartridge or the cartridge system, wherein the content of the container is thus expelled from the container.

23. The method according to one of Claims 20 to 22, **characterised in that**
the cross section of a connection of the compressed gas container to the holder, and thus a pressure acting on the container, is adjusted by an operating facility, in particular a throttle valve.

24. The method according to one of Claims 20 to 23, **characterised in that**
the holder is rotated or displaced up to a stop or a locking means.

25. The method according to one of Claims 20 to 24, **characterised in that**
the opening means is shifted linearly with respect to the compressed gas container.

26. Use of a dispensing device according to one of Claims 1 to 19, in particular with application of a method according to one of Claims 20 to 25, for pressing out pasty one-component polymethylmethacrylate bone cements, pasty two-component polymethylmethacrylate bone cements, pasty three-component polymethylmethacrylate cements, dental impression materials, inorganic bone cements and/or polymethylmethacrylate bone cements, preferably by mixing a powder component and a liquid monomer component.

## Revendications

1. Dispositif de décharge pour extruder le contenu d'un récipient par une application à l'action d'un gaz comprimé, comprenant un boîtier (2, 32, 62), au moins un récipient de gaz sous pression (16, 46, 76), un dispositif d'actionnement (10, 11), pour l'ajustement d'un flux gazeux provenant du récipient de gaz sous pression (16, 46, 76), et un moyen d'ouverture (12, 42, 72), pour l'ouverture du récipient de gaz sous pression (16, 46, 76), où le moyen d'ouverture (12, 42, 72) est mobile par rapport au récipient de gaz sous pression (16,46,76) et le dispositif de décharge (1) comprend un support (3, 33, 63) pour la fixation d'un récipient sur le dispositif de décharge (1), **caractérisé en ce que**
le support (3, 33, 63) est en communication avec le moyen d'ouverture (12, 42, 72) de telle manière que, par la fixation d'un récipient sur le support (3, 33, 63), un mouvement du moyen d'ouverture (12, 42, 72) par rapport au récipient de gaz sous pression (16, 46, 76) se produit, qui ouvre le récipient de gaz sous pression (16, 46, 76).

2. Dispositif de décharge selon la revendication 1, **caractérisé en ce que**
le récipient de gaz sous pression (16, 46, 76) comprend au moins une cartouche de gaz (16, 46), notamment au moins une cartouche de CO₂, où au moins une des cartouches de gaz (16, 46) peut être ouverte par le moyen d'ouverture (12, 42, 72) et le moyen d'ouverture (12, 42, 72) peut être déplacé dans la direction longitudinale de la cartouche de gaz (16, 46).

3. Dispositif de décharge selon les revendications 1 ou 2, **caractérisé en ce que**
le dispositif de décharge présente un récipient et le récipient est une cartouche ou un système de cartouches avec plusieurs cartouches, notamment disposées parallèlement, où la cartouche ou le système de cartouches contiennent de préférence un ciment osseux, de manière particulièrement préférée, un ciment osseux multi-composant.

4. Dispositif de décharge selon la revendication 3, **caractérisé en ce**
**qu'**au moins un piston de transport est agencé, mobile à l'intérieur, dans la cartouche ou dans le système de cartouches, qui peut être mis en marche par la pression du gaz provenant du récipient de gaz sous pression (16, 46, 76).

5. Dispositif de décharge selon l'une des revendications précédentes, **caractérisé en ce que**
le récipient de gaz sous pression (16, 46, 76) peut être mis en communication avec une ouverture dans le support (3, 33, 63) par l'intermédiaire d'au moins une conduite de gaz sous pression (9, 39, 69), où le dispositif d'actionnement (10, 11) est agencé dans la connexion réalisée par la conduite de gaz sous pression (9, 39, 69) ou les conduites de gaz sous pression (9, 39, 69).

6. Dispositif de décharge selon la revendication 5, **caractérisé en ce que**
la conduite de gaz sous pression (9, 39, 69), ou les conduites de gaz sous pression (9, 39, 69), comprend, ou comprennent, au moins une partie souple, notamment, un bout de tuyau, ou qu'au moins un tuyau souple est installé.

7. Dispositif de décharge selon l'une des revendications précédentes, **caractérisé en ce que**
le dispositif d'actionnement (10, 11) comprend une soupape d'étranglement (10) réglable qui peut de préférence être actionnée par une gâchette (11).

8. Dispositif de décharge selon l'une des revendications précédentes, **caractérisé en ce que**
le dispositif de décharge (1) comprend une poignée (11), de préférence une poignée pistolet (18), sur laquelle, de préférence, est agencée la gâchette (11) pour actionner le dispositif d'actionnement (10, 11).

9. Dispositif de décharge selon l'une des revendications précédentes, **caractérisé en ce que**
le moyen d'ouverture (12, 42, 72) comprend un mandrin (14, 44, 74) avec lequel le récipient de gaz sous pression (16, 46, 76) peut être mis en perce, où une connexion étanche en pression peut être réalisée avec le récipient de gaz sous pression (16, 46, 76) par l'intermédiaire du mandrin (14, 44, 74) et le mandrin (14, 44, 74) est de préférence relié avec la conduite de gaz sous pression (9, 39, 69) ou les conduites de gaz sous pression (9, 39, 69).

10. Dispositif de décharge selon l'une des revendications précédentes, **caractérisé en ce que**
le support (63) est disposé mobile par rapport au récipient de gaz sous pression (76) et le moyen d'ouverture (72) est solidement relié avec le support (63), où un récipient peut être inséré dans le support (63) et le support (63) comprend un organe de butée (79) à cet effet, qui se met en prise avec un organe de butée complémentaire d'un récipient, et où le récipient de gaz sous pression (76) s'ouvre par la force s'exerçant sur le support (63) pour la fixation d'un récipient dans le support (63) du dispositif de décharge (1).

11. Dispositif de décharge selon la revendication 10, **caractérisé en ce que** le récipient de gaz sous pression (76) est solidement relié, ou peut être relié, avec le boîtier (62) et le support (63) est disposé avec le moyen d'ouverture (72) mobile linéairement par rapport au boitier (62) sur le boitier (52).

12. Dispositif de décharge selon l'une des revendications 1 à 9, **caractérisé en ce**
**qu'**un récipient peut être rentré en vissant ou vissé dans le support (3, 33) et le support (3, 33) est disposé en pouvant tourner par rapport au moyen d'ouverture (12, 42), où un filetage (38) est agencé sur le support (3, 33), notamment sur un tuyau (6, 36) sur le support (3, 33) qui s'étend vers l'intérieur du boitier (2, 32), filetage qui s'engrène avec le moyen d'ouverture (12, 42), ou une came (8) est disposée qui se met en prise avec une pièce complémentaire sur le moyen d'ouverture (12, 42), et le moyen d'ouverture (12, 42) est disposé mobile linéairement contre le support (3, 33) et le récipient de gaz sous pression (16, 46), de sorte qu'une rotation du support (3, 33) provoque un mouvement linéaire du moyen d'ouverture (12, 42) contre le récipient de gaz sous pression (16,46) et ainsi l'ouverture du récipient de gaz sous pression (16, 46) lors de la fixation d'un récipient sur le support (3, 33).

13. Dispositif de décharge selon la revendication 12, **caractérisé en ce que**
le récipient de gaz sous pression (16,46) est solidement relié, ou peut être relié, avec le boitier (2, 32) et le support (3, 33) est disposé sur le boitier (2, 32) avec le moyen d'ouverture (12, 42) pouvant tourner par rapport au boitier (2, 32), de préférence, pouvant tourner d'un angle entre 5 ° et 360 °, de manière particulièrement préférée, entre 5 ° et 15 °ou entre 180 ° et 360 °, où le mouvement de rotation du support (3, 33) est notamment limité par une butée et/ou un arrêt.

14. Dispositif de décharge selon les revendications 12 ou 13, **caractérisé en ce**
**qu'**un moyen de fixation (19, 39) est agencé sur le support (3, 33) pour la fixation d'un récipient sur le support (3, 33), notamment un filetage intérieur (49), un fermeture par baïonnette ou par bouchon (19), qui forment un filetage.

15. Dispositif de décharge selon les revendications 12 à 14, **caractérisé en ce que**
dans la position de base du support (3, 33) sans récipient, le filetage (38) ou la came (8) fixent le moyen d'ouverture (12, 42) et le récipient de gaz sous pression (16, 46) est fermé, et dans une position tournée du support (3, 33) avec un récipient qui y est fixé, le filetage (38) ou la came (8) fixent le moyen d'ouverture (12, 42) de telle manière que le récipient de gaz sous pression (16, 46) est relié avec une ouverture (7, 37) dans le support (3, 33) laissant passer le gaz.

16. Dispositif de décharge selon l'une des revendications précédentes, **caractérisé en ce que**
le récipient de gaz sous pression (16, 46, 76) est relié, ou peut être relié, avec une fixation (12, 42, 72) sur le boitier (2, 32, 62) en pouvant être détaché, notamment à l'intérieur du boitier (2, 32, 62),de préférence par l'intermédiaire d'un filetage, de manière particulièrement préférée, par un filetage interne sur la fixation (12, 42, 72) et une filetage complémentaire, de manière particulièrement préférée, un filetage externe au récipient de gaz sous pression (16, 46, 76).

17. Dispositif de décharge selon l'une des revendications précédentes, **caractérisée en ce que**
l'ouverture du récipient de gaz sous pression (16, 46, 76) s'effectue de manière synchronisée avec l'insertion d'un récipient dans le dispositif de décharge (1).

18. Dispositif de décharge selon l'une des revendications précédentes, **caractérisé en ce**
**que**, sur le support (3, 33, 63), sont disposés une ouverture (7, 37, 67) et un tuyau cylindrique (6, 36, 66), qui débouche dans l'ouverture (7, 37, 67) et qui peut être mis en rotation autour de son axe ou peut se déplacer le long d'un axe logé contre le récipient de gaz sous pression (16, 46, 76), où le support (3, 33, 63) est logé en pouvant tourner ou se déplacer de préférence dans une partie du boitier (2, 32, 62) conçue comme une gaine.

19. Dispositif de décharge selon l'une des revendications précédentes, **caractérisé en ce**
**qu'**au moins un anneau d'étanchéité (4, 34, 64) ou un disque d'étanchéité (4, 34, 64) sont disposés sur le support (3, 33, 63), avec lesquels, ou par lesquels, un récipient peut être relié de manière étanche au support (3, 33, 63) avec le dispositif de décharge (1).

20. Procédé pour l'activation d'un dispositif de décharge, notamment selon l'une des revendications précédentes, dans lequel un récipient qui contient un matériau à extruder, est inséré dans un support du dispositif de décharge, **caractérisé en ce que**
la force à exercer lors de l'insertion du récipient est utilisée pour faire tourner et/ou déplacer le support,
par la rotation ou le déplacement du support, un moyen d'ouverture est déplacé contre un récipient de gaz sous pression du dispositif de décharge et
le récipient de gaz sous pression est ouvert par le mouvement du moyen d'ouverture.

21. Procédé selon la revendication 20, **caractérisé en ce que** le gaz sortant du récipient de gaz sous pression est emmené vers le support et exerce une pression sur au moins une partie du récipient.

22. Procédé selon la revendication 21, **caractérisé en ce qu'**un piston de transport dans le récipient, notamment dans la cartouche ou dans le système de cartouches, est propulsé par la pression, où le contenu du récipient est expulsé du récipient.

23. Procédé selon l'une des revendications 20 à 22, **caractérisé en ce que** la section transversale d'une liaison du récipient de gaz sous pression vers le support et, en conséquence, la pression s'exerçant sur le récipient, est réglée par un dispositif d'actionnement, notamment une soupape d'étranglement.

24. Procédé selon l'une des revendications 20 à 23, **caractérisé en ce que** le support est tourné ou déplacé jusqu'à une butée ou un arrêt.

25. Procédé selon l'une des revendications 20 à 24, **caractérisé en ce que** le moyen d'ouverture est déplacé linéairement contre le récipient de gaz sous pression.

26. Utilisation d'un dispositif de décharge selon l'une des revendications de 1 à 19, notamment moyennant l'emploi d'un procédé selon l'une des revendications 20 à 25, pour l'extrusion de ciments osseux de polyméthylméthacrylate monocomposant de forme pâteuse, de ciments osseux de polyméthylméthacrylate bicomposant de forme pâteuse, de ciments osseux de polyméthylméthacrylate tricomposant de forme pâteuse, de masses de moulage dentaire, de ciments osseux inorganiques et/ou de ciments osseux de polyméthacrylate, de préférence, par un mélange d'un composant en poudre et d'un composant monomère liquide.
